# EUROPEAN PATENT SPECIFICATION

(11) **EP 1 820 478 B1**
(45) Date of publication and mention of the grant of the patent: **28.04.2010**
(21) Application number: 07102564.7
(22) Date of filing: 16.02.2007
(51) Int. Cl.: A61F 5/44, A61M 39/10

(54) **A collection bag arrangement comprising a hose connection**
Anordnung aus Sammelbehälter und Schlauchverbindung
Sac de collecte comprenant un raccord de tuyau

(30) Priority: 16.02.2006 DK 200600222; 27.04.2006 DK 200600600
(43) Date of publication of application: 22.08.2007
(73) Proprietor: Unomedical A/S, 3460 Birkerød (DK)
(72) Inventor: James, Michael, Elkesley Nottinghamshire DN22 8AP (GB); Hansen, Trygve Kalf, 4040 Jyllinge (DK)
(74) Representative: Plougmann & Vingtoft A/S

(56) References cited:
- EP-A1- 0 633 039
- WO-A-89/05119
- FR-A1- 2 742 518
- US-A- 2 198 922
- US-A- 4 758 023
- US-A1- 2004 215 158

## Description

The present invention relates to a hose connection of an arrangement for collection of liquid from a patient. In particular, the connection may be used between a collection bag and a vessel for measuring the volume of urine from bedridden patients.

### BACKGROUND OF THE INVENTION

Measurement and collection of liquid from patients at hospitals is obtained by use of a catheter inserted into the patient, e.g. into the bladder, said catheter being connected to a measuring vessel or a collection bag for collecting the liquid.

Often the vessel or catheter is fixed connected to a collection bag, which can be emptied by use of a valve in the bottom of the bag. However, it is preferable that the bag can be replaced by a new one when it is full instead of just emptying it. This replacement must be done more than once a day, and it is therefore important that it can be done fairly easily for the nurse in order to reduce his/her workload and to avoid the risk of spillage. Thus, the connection between the bag to be replaced and the other part(s) must be separable and performed without the need of much energy, but it still has to be a proper connection and be completely tight during use.

The characteristics of known hose connections are that they are either to loose and disconnect during use, or are too tight and very difficult to disconnect.

US 2004/0215158 discloses a collection bag arrangement comprising an in-line hose connector for a urinary catheter according to the preamble of claim 1. The connector has a first part with a hose barb connected to the catheter tube and a second part with a cylindrical opening and a circumferentially extending fastening strap. A projection of the second part fits into a hole on the fastening strap after twisting the pieces and is so shaped as to aid in disengaging the fastening strap from the projection and thus disconnect the parts when a tension force is exerted.

As the connector parts of US 2004/0215158 are both made of a hard plastic, an O-ring between these parts must provide the sealing. Furthermore, the construction is very complicated and expensive as it consists of two moulded parts each having a part of a catheter tube in the end.

Another known hose connection consists of a female tube of a bag being slid over a male tube, the male tube having a plurality of conical shaped protrusions with an increasing diameter so that the male tube is connected to the female tube by means of friction provided due to the increasing diameter. Such a connection provides a good tightening, but it is very difficult to separate without using much energy and without spillage, or it may even not be separable at all.

Other hose connections are disclosed in US 2002/123739, EP 0 901 778, DE 298 18 311, US 5,356,396, and US 4,579,126.

It is an object of the present invention to provide collection bag arrangement comprising a hose connection between a collection bag and the fluid connection to the patient, which connection is easy to assemble and separate, simpler and thus cheaper to manufacture, while still providing proper tightening during use.

### SUMMARY OF THE INVENTION

This objective and the advantages that will become evident from the following description of the invention are obtained by the collection bag arrangement defined in claim 1.

The second tubular member is preferably connected to the bag by adhesion, but it may be an integrated part of the bag, e.g. by in-moulding. The second tubular member can for example be a piece of a known soft plastic hose, e.g. of PVC, which can be adhered in a prefabricated hole in the bag, thus providing a cheap construction being easy to manufacture. As the construction is cheap to manufacture, it allows for a disposable bag that does not need be reused but can be thrown away after use.

The protrusion(s) comprise a support surface for the recess(es) or edges, which extends substantially perpendicularly and radially out from the outer surface, and an inclined surface extending from the distal end of said support surface towards the outer surface. The support surface is at least partly formed as a ledge which supports the recess(es) which preferably has a corresponding surface adapted to abut on the ledge in order to provide a safely locked connection. The ledge is level or flat provides a rest which the corresponding recess(es) may engage with for preventing unintended disconnection caused by tension force.

The inclined surface provides a chute along which the tubular member can slide until the recess reaches the support surface, the chute enabling an easier connection between the two tubular members. The inclined surface may for example be convex or concave.

Preferably, the recesses go through the sidewall of the second member and have an outline substantially identical to the outline of the protrusions, such as, but not limited to, half-circle formed or arrow-formed or rectangular.

The second tubular member may comprise slot(s) providing an opening channel in the sidewall extending from an upper edge of the second member to the recess(es), so that the protrusions can be guided through the channel to obtain the engaged position in the recess(es).

The first and second tubular members may comprise two recesses and protrusions, respectively, arranged on opposite sides of each member. In an embodiment, the first tubular member comprises two protrusions arranged diametrically opposite. However, the tubular members may comprise just one or more than two recess(es) and protrusions, respectively, being evenly or unevenly distributed along the surface of the members.

The first tubular member is preferably substantially conical with an outer diameter (d3) near the outlet being smaller than an outer diameter (d1) upstream thereof, and an inner diameter (d4) of the second tubular member being preferably larger than the outer diameters (d1) and (d3) of the first tubular member. The second tubular member is thereby easy to slide onto the first tubular member without any significant friction, but will still be locked connected to the first tubular member due to the engagement between the recess(es) and protrusion(s).

In combination with the engagement between the recess(es) and protrusions, a connection is provided which is easy to assemble and separate, simple and cheap to manufacture and provides a proper tightening.

In order to obtain an even better tightening, the first tubular member may comprise an intermediate widened portion with a diameter (d2) being larger than (d1), (d3) and (d4). However, the diameter (d2) is preferably not much larger than (d4) in order to obtain the easy assembling and separation without much friction. The widened portion may be provided by a conical protrusion on the outer surface.

The diameters may for example be (d1) = 11 mm, (d2) = 11.4 mm, (d3) = 10.8 mm, and (d4) = 11.2 mm, but they can of course vary.

Preferably, the first and second tubular member have congruent cross-sectional outlines meaning that they fit smoothly into each other, and the second member preferably covers the entire outer surface of the first tubular member, but not necessarily.

In an embodiment, the protrusions are provided at a distal end of the first tubular member and are adapted to engage a distal edge of the second tubular member. In one embodiment, the first tubular member comprises at least two opposite flexible leg parts each having a protrusion (a barb) adapted to engage a distal edge of the second tubular member, the leg parts being bendable towards each other for disengaging the protrusions from the edge of the second tubular member.

The first and second tubular members can be connected by pushing and/or turning the second tubular member over the first tubular member until the protrusions engage the recesses. In the embodiment with the opening channel as mentioned above, the second tubular member is both pushed and turned in order to guide the protrusions into the recess(es). The protrusions and recesses can be disengaged from each other preferably by compressing and/or turning the second tubular member. The second tubular member is preferably made of a soft plastic material, so that it can be compressed on two opposite sides resulting in a widening of the sidewall containing the recess(es), which then will disengage the protrusions on the first tubular member.

According to the invention, the first and/or the second tubular members comprise a substantially semicircular or annular section forming a ramp engaging with the other member for facilitating disengagement of the members by twisting them in relation to each other. Preferably it is the second tubular member that is twisted. The semicircular section forming a ramp is preferably provided so as to partly encircle a part of the first tubular member having the diameter d1 and preferably closer to the inlet end of the first tubular member than to the outlet end.

In embodiments the first and/or second tubular member may alternatively comprise an annular section at least partly forming a ramp. By at least partly is meant that the annular section may e.g. partly comprise a substantially semicircular section forming a ramp and partly an adjacent semicircular section without a ramp. However, the annular section may preferably comprise two adjacent semicircular sections provided with ramps. In principle any number of sections provided with ramps may together form the annular section. The engaging surface on the other member may in such embodiments be suitably adapted according to the section(s) on the first member.

The semicircular or annular section forming a ramp may be provided on, or as, the engaging surface of the second tubular member provided that one or more corresponding recesses are made in the outer engaging surface part of the first tubular member.

Preferably, only one of the members is provided with a semicircular or annular section but such a section may also be provided on both members.

By providing such a ramp on either one or both members according to the invention, it is particularly easy for a person to intentionally disconnect the hose connection, preferably by twisting the second tubular member in relation to the first tubular member. This is because the ramp on the section slides against the surface of the other member which in turn causes the recess(es) on the second tubular member to be released from its resting position on the ledge of the first tubular member. No excessive force will thus be necessary; the members can be released from each other in a comfortable manner without compromising a safe and tightly locked connection in use.

The first tubular member is preferably made of a hard plastic material having a Shore A value of approx. 100, such as SAN plastic. The second tubular member is preferably made of a soft plastic material having a Shore A value of approx. 80, such as poly-vinyl-chloride.

The first tubular member may be a part of a hose/catheter connected directly to an organ of a patient, or it may be a part of a measuring vessel for measuring volume of liquid, such as urine. The first tubular member may be connected to this vessel, or it may be a fixed in-moulded part of said vessel defining the outlet of the vessel.

According to a second aspect and as defined in claim 18, the invention relates to an arrangement for measuring the volume of liquid from a patient, the arrangement comprising a measuring vessel and a collection bag being connected by a hose connection according to the above described hose connection.

According to a third aspect, the invention relates to a use of an arrangement according to the second aspect for collection of urine from a patient.

Moreover, a vessel for measuring volume of liquid, in particular urine from bedridden patients is disclosed. The vessel comprises a hollowed member for receiving and containing the liquid, the bottom surface of the member having a curved shape provided on each side of an intermediate part defining a central column terminating in an outlet of the vessel.

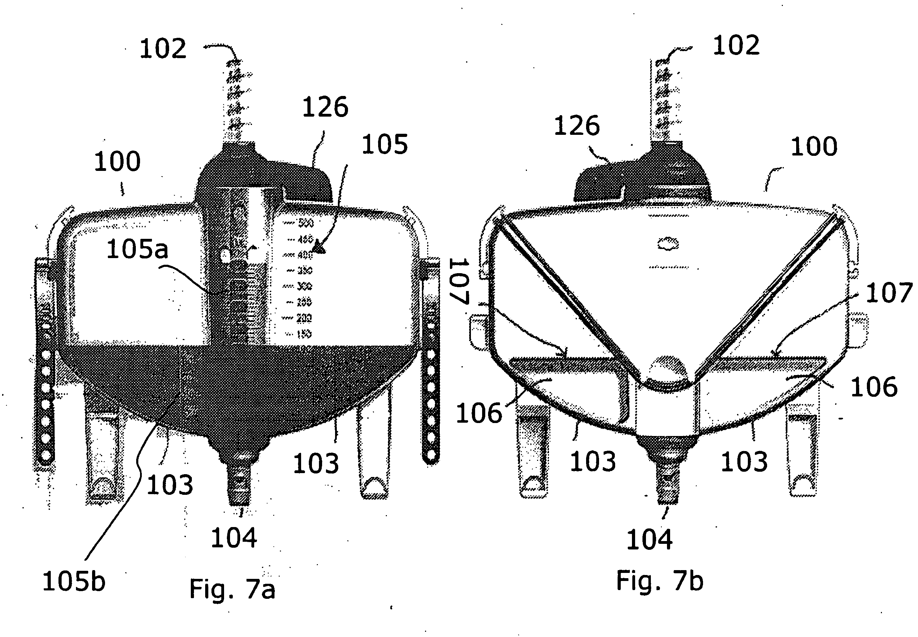

The outlet can for example be an outlet defined by the first tubular member according to the connection as mentioned above, so that the vessel can be coupled to the bag by a hose connection as mentioned above (see Figs. 7a+b).

The vessel is provided with a measuring scale indicating the volume of liquid present therein. As the bottom surface is curved, the scale can basically not be a linear scale, but in order to solve that, the vessel is provided with recesses or other elements taking up a predefined volume of the vessel, which makes it possible to use a linear scale on the vessel. The recesses can for example be provided on the backside of the vessel and defines a straight horizontal bottom surface inside the vessel at a level substantially equal to the level at which the curved surface begins.

The curved surface of the vessel allows for a better hygiene as there are no corners that can be difficult to access and clean from outside. Furthermore, the curved surface provides a more ergonomic vessel being easier to hold.

An embodiment of the vessel is shown in Figs. 7a-8b.

The invention may be used as an arrangement for measuring and collecting body liquids, the arrangement comprising a measuring vessel having at its top end a liquid inlet and at its lower end a liquid outlet provided with a valve and a liquid collection bag connected to the liquid outlet and suspended from the measuring vessel, a hollow valve body being placed in and vertically displaceable within the measuring vessel, and a part of the liquid outlet has the shape of a valve seat for the hollow valve body, the interior of the valve body being divided into a reception chamber and an overflow chamber, said reception chamber being connected to the liquid inlet and having at the lower end of the valve body at least one outlet opening which in the closed position of the valve body is closed by the valve seat and which at its upper part is connected to the measuring vessel through at least one hole in the chamber wall, and said overflow chamber having at its lower end a duct which is directly connected to the liquid outlet of the measuring vessel and which at its top end is connected to the measuring vessel via a hole in the chamber wall, the holes connecting the reception chamber to the measuring vessel and the measuring vessel to the overflow chamber being placed on the same side of the hollow valve body, and wherein the measuring vessel and collection bag is connected by a hose connection according to the first aspect of the invention.

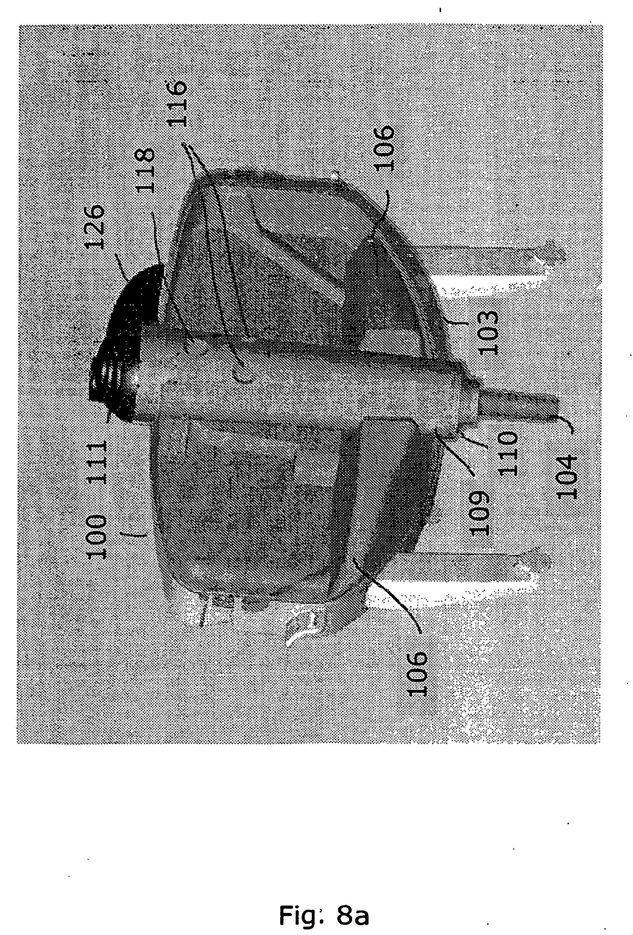

The arrangement is further described below with reference to Figs. 8a-9e.

### DESCRIPTION OF THE FIGURES

A hose connection of the type forming part of the invention as claimed is only shown in Figs. 10a to 10c.

Embodiments of connections will now be described in details with reference to the other accompanying figures, wherein
Figs. 1a-c show a first embodiment of a connection,
Figs. 2a-c show a second embodiment of a connection,
Figs. 3a-c show a third embodiment of a connection,
Figs. 4a-c show a fourth embodiment of a connection,
Figs. 5a-c show a fifth embodiment of a connection,
Fig. 6 shows a cross sectional view of a first tubular member,
Figs. 7a-b show a vessel,
Fig. 7c shows a vessel and a collection bag connected by a hose connection,
Fig. 8a-b show the vessel of Figs. 7a-c more in detail,
Fig. 9a-e show sectional views of the valve body of the vessel.
As mentioned above, Fig. 10a-c show schematic, perspective views of a first tubular member having a semicircular section provided with a ramp according to the invention.

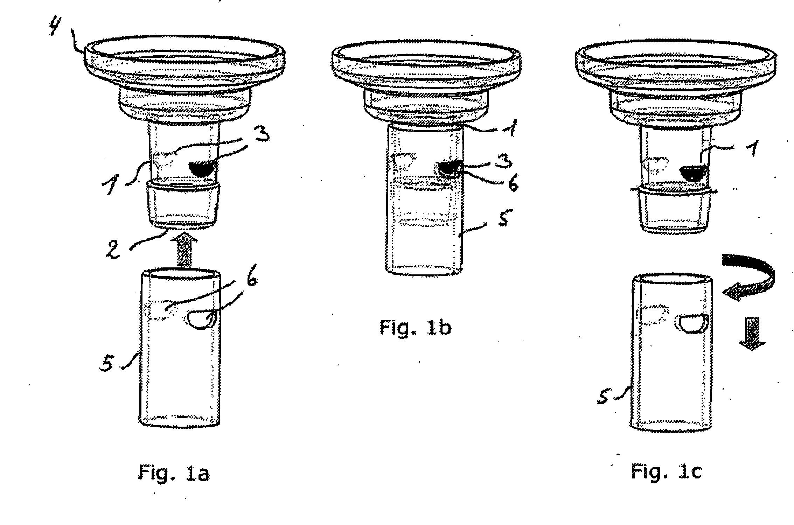

Figs. 1a-c show a first embodiment of a connection comprising a first tubular member 1 defining an outlet 2 and having two oppositely arranged protrusions 3 provided on its outer surface. The first tubular member 1 forms part of a bottom part 4 of e.g. a vessel.
A second tubular member 5 is adapted to be connected to the first tubular member 1 by sliding it over the first tubular member 1 so that the two oppositely arranged recesses 6 engage with the respective protrusion 3 as shown in Fig. 1b.

In order to separate the connection, the second tubular member 5 is turned and subsequently drawn downwards as shown in Fig. 1c so that the protrusions 3 disengage from the recesses 6.

As the second member 5 is preferably made of a soft plastic material, it may be compressed on the two sides opposite to the sides where the recesses 6 are provided, so that the sides comprising the recesses are widened and can disengage from the protrusions 3.

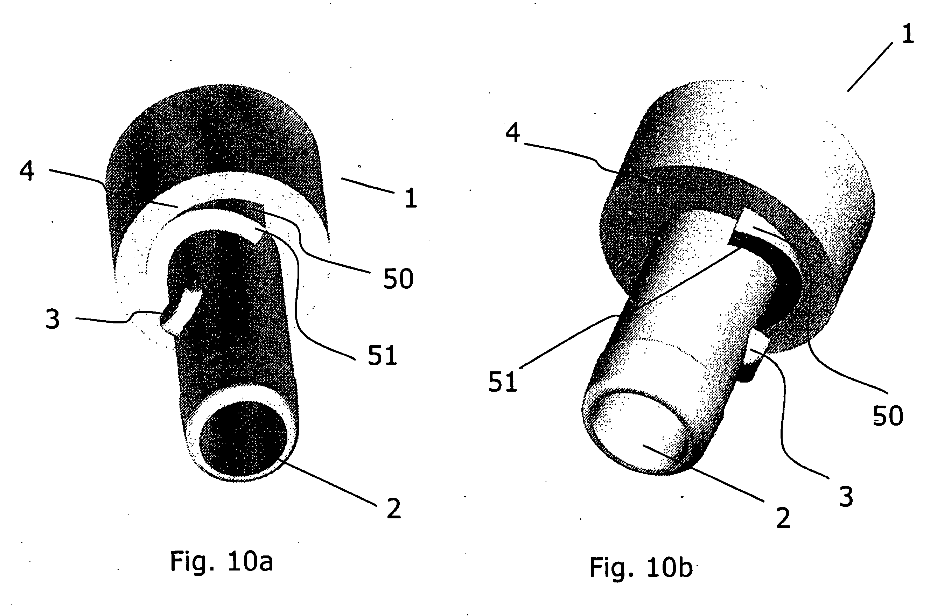

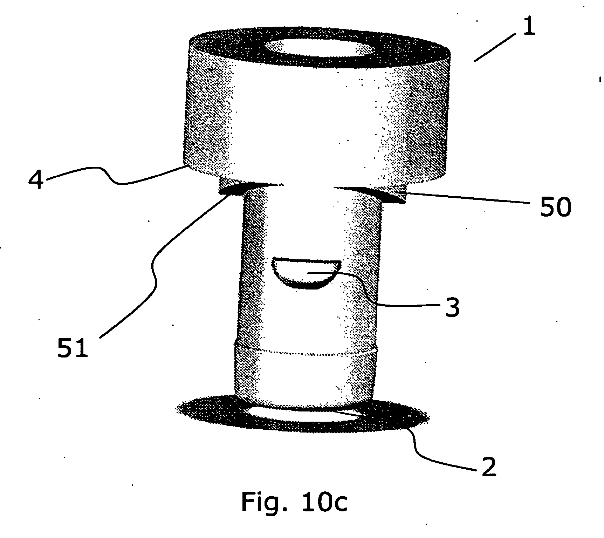

The protrusion 3 comprises an upper support surface at least partly formed as a ledge supporting a corresponding support surface of the recesses 6 of the second tubular member, so that the second tubular member 5 is positioned in a locked but separable connection with the first tubular member, as best shown in Fig. 1b. The support surface extends perpendicularly and radially out from the first tubular member thereby constituting the ledge. This ensures that the second tubular member does not come off by accident, as the support surface of the recess rest on the ledge of the protrusion 3. By use of this connection, it is easy to connect the two tubular members with each other without much energy needed, while still obtaining a safe locked connection due to the protrusions 3 and recesses 6. Furthermore, the connection is easily separable, as the second tubular member just need to be twisted or turned and pulled in order to disengage the protrusions 3 from the recesses 6. In other embodiments, indicated in figures 10a-c, the easy separation or disconnection of the members is facilitated by providing either one, or both, of the tubular members with a ramp.

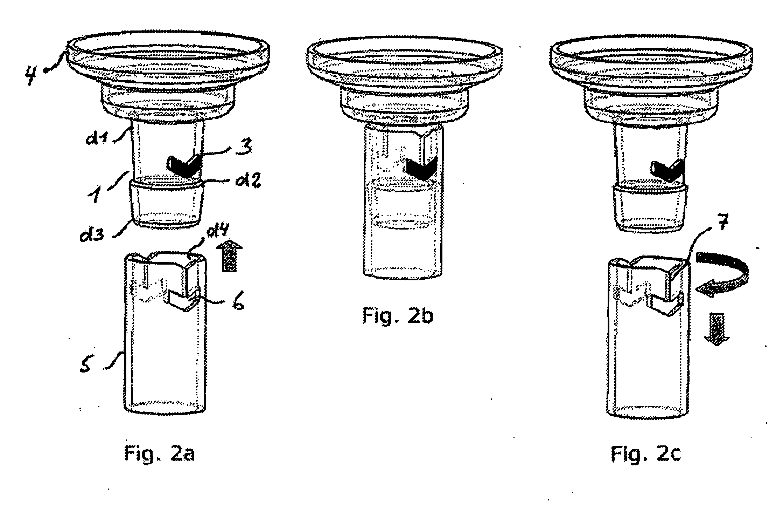

Figs. 2a-c show another embodiment of the connection, wherein the protrusions 3 are formed as an arrow, the recesses 6 having a corresponding shape. The second tubular member 5 is connected to and separated from the first tubular member 1 in a similar way as the embodiment of Figs. 1a-c.

The diameters d1, d2, d3 and d4 as referred to in the specification above are shown in Fig. 2a.

The second tubular member 5 is slotted along the line 7 to ease the mounting.

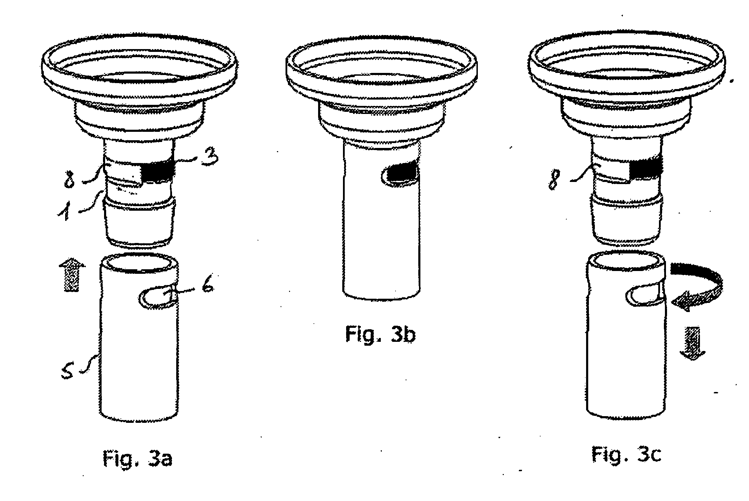

Figs. 3a-c show another embodiment of the connection having elongated protrusions 3 extending peripherally along the outer surface of the first tubular member 1. The second tubular member 5 is connected to and separated from the first tubular member 1 in a similar way as the embodiments of Figs. 1a-2c.

Recesses 8 are provided in the outer surface of the first tubular member in order to ease the mounting, but can also be used as space for compressing the second tubular member 5 when separating it from the first tubular member 1.

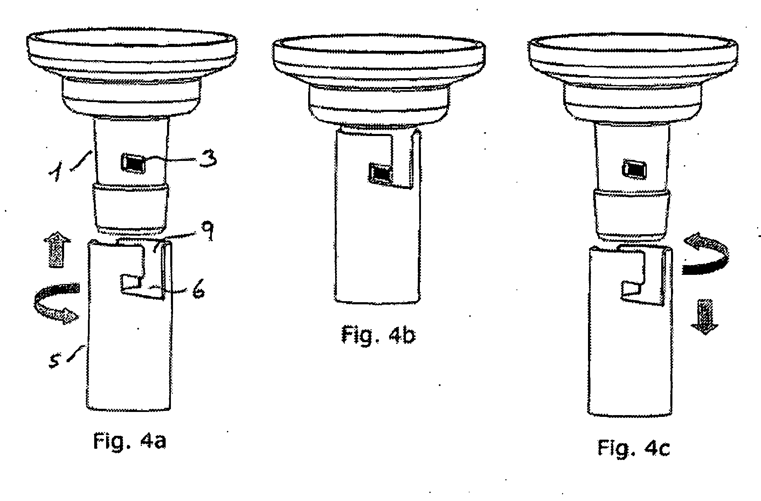

Figs. 4a-c show another embodiment of the connection according to the invention, wherein the second tubular member 5 is slid on and finally turned in order to connect it to the first tubular member 1. The first member 1 comprises a slot 9 providing an opening channel in the sidewall extending from an upper edge of the member to the recesses 3. The protrusions 3 are guided through the channel to obtain the engaged position in the recesses 6. The protrusions 3 and recesses 6 have corresponding slanting upper support surfaces for obtaining an even better connection not separating by accident.

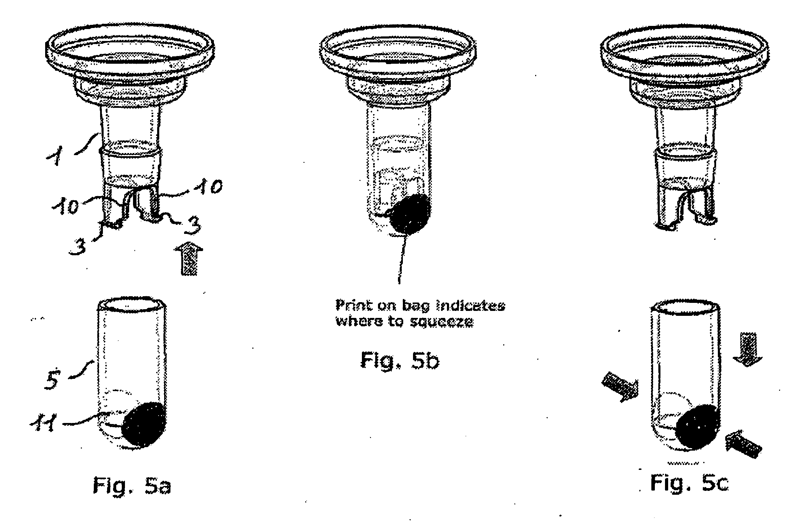

Figs. 5a-c show another embodiment of the connection according to the invention, wherein the protrusions (barbs) 3 are provided at a distal end of the first tubular member 1 the protrusions 3 being adapted to engage a distal edge 11 of the second tubular member 5. The first tubular member 1 comprises two opposite flexible leg parts 10 each having a protrusion 3, the leg parts 10 being bendable towards each other for disengaging the protrusions 3 from the edges of the second tubular member 5. The second tubular member 5 may for example comprise prints showing where to squeeze the leg parts 10, as shown in Fig. 5b.

Thus, the second tubular member 5 is separated from the first tubular member 1 by squeezing the leg parts 1 towards each other and then drawing the member 5 downwards, as shown in Fig. 5c.

The outline of the protrusions and recesses shown in figures 1a-5b are examples, which provide a proper locked separable hose connection, but other outlines may of course be used.

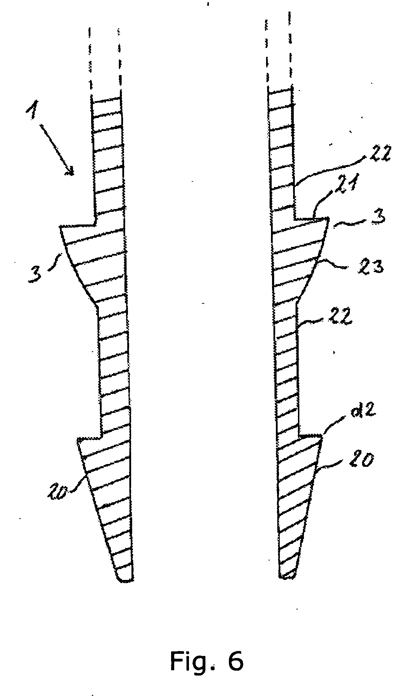

Fig. 6 shows a cross sectional view of a first tubular member 1 according to the invention. The member 1 comprises a widened conical portion 20 with a diameter d2 as shown in Fig. 2a and a protrusion 3. The protrusion 3 comprises a support surface 21 for a corresponding support surface of the recesses of a second tubular member, the support surface 21 extending substantially perpendicularly and radially out from the outer surface 22. An inclined surface 23 extends from the distal end of said support surface 21 towards the outer surface. The support surface 21 is shown in the form of a substantially even or plane ledge. The inclined surface 23 eases the sliding movement of a second tubular member over the protrusion 3 to obtain the locked but separable connection between the ledge on the first and the corresponding recess on the second tubular member.

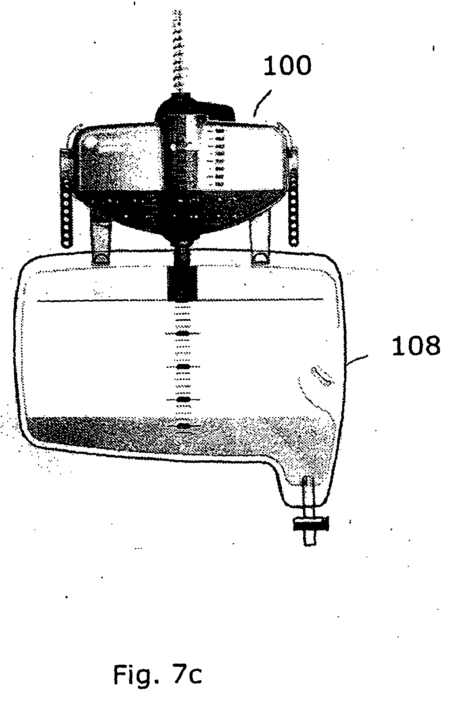

Figs. 7a-b show a front and back view of a vessel according to the invention for measuring volume of liquid, in particular urine from bedridden patients. The vessel 100 comprises a hollowed member with an inlet 102 for receiving and containing the liquid, and wherein the bottom surface 103 has a curved shape. An outlet 104 is provided in the bottom of the vessel 100, which can be coupled to a collection bag as shown in Fig. 7c.

The vessel 100 is provided with a measuring scale 105 indicating the volume of liquid present therein. On the basis of a curved bottom surface 103, the measuring scale 105 can basically not be a linear scale, but in order to solve that problem, the vessel 100 is provided with filling elements 106 provided as recesses on its backside defining a straight horizontal bottom surface 107 at a level substantially equal to the level at which the curved surface begins. Due to the presence of the filling elements 106 taking up a predefined volume of the vessel and thereby compensating for the curved bottom surface 103, it is possible to use a linear scale 105.

The curved surface 103 of the vessel 100 allows for a better hygiene as there are no corners that can be difficult to access and clean from outside. Furthermore, it provides a more ergonomic vessel being easier to hold.

Fig. 7c shows a vessel 100 and a collection bag 108 connected to each other via the outlet 104 of the vessel and an inlet of the bag, which may be formed by a tubular member being adhered to the bag 108.

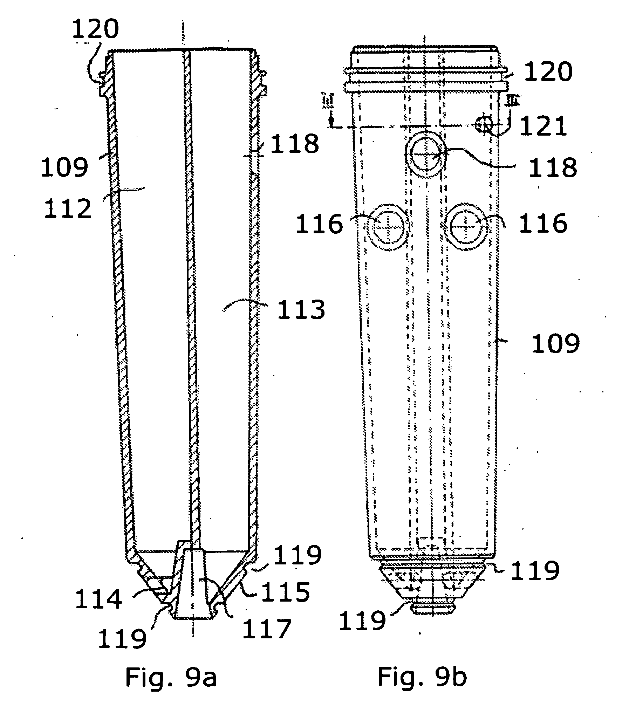

Figs. 8a-b show transparent views of the vessel of Figs. 7a-c in order to see the inside of the vessel more in detail. The vessel 100 comprises the hollowed member for receiving and containing the liquid, and the bottom surface 103 has a curved shape provided on each side of a central column terminating in the outlet 104 of the vessel 100. The central column defines a valve body 109 for the vessel, the valve body 109 being axially displaceable by rotation and which at its lower end is in contact with a valve seat 110 and is closed at the top with a cover 111. The valve body 109 is described more in detail with reference to Figs. 9a-e.

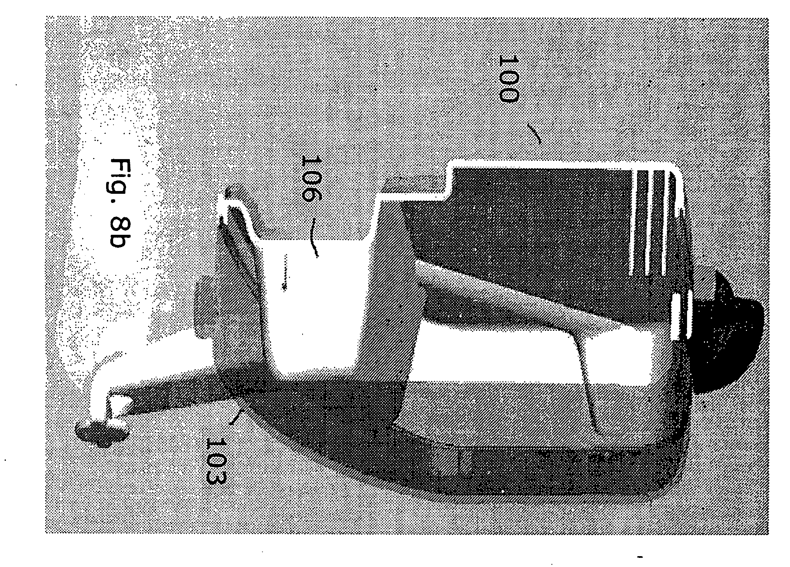

Fig. 8b is a cross-sectional view of the vessel 100. As seen in fig. 8b, the filling element 106 is provided as a projection moulded in the back wall of the vessel.

Figs. 9a-e show sectional views of a valve body 109 of the vessel 100 as shown in Figs. 7a-c and Figs. 8a-b.

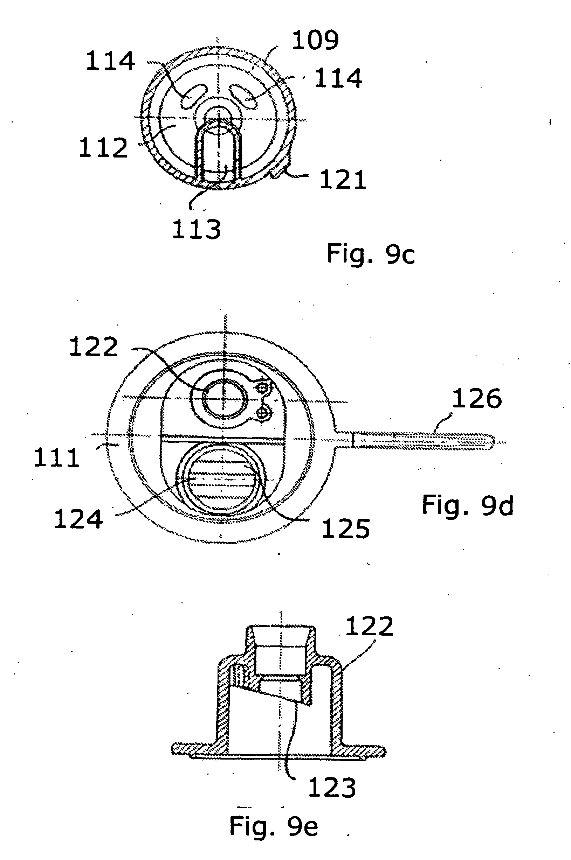

Fig. 9c is a sectional view along the line III-III of the valve body of Fig. 9a, Fig. 9d shows the upper part of the valve body seen from the bottom, and Fig. 9e shows the upper part of the valve body of Fig. 9d in sectional view.

The central column defines the valve body 109, which is constructed with two chambers; a reception chamber 112 and an overflow chamber 113. At its lower end the reception chamber 112 is provided with two holes 114 ending in a valve surface 115 which contacts the valve seat 110 in its closed position thus preventing liquid outflow. At the upper end of the reception chamber 112 two holes 116 are provided in the wall separating the chamber 112 from the surrounding vessel 100.

At its lower end the overflow chamber 113 is provided with a duct 117 through which said overflow chamber 113 is in direct contact with the liquid outlet of the vessel. The liquid outlet is defined by a tubular member so that the vessel can be connected to a collection bag 108.

At the top end of the overflow chamber 113 a hole 118 is provided in the valve wall.

Two annular-shaped grooves 119 each comprising an O-ring (not shown) are provided on the outside of the valve body at the lower end thereof. An annular groove 120 comprising an O-ring (not shown) is provided.

The outside of the valve body 109 is also provided with a projection 121 being inserted in a helical groove formed on the outside of the back wall of the measuring vessel 100.

The cover 111 of the valve body 109 is provided with two openings one being a tubular member 122 ending in an inclined surface 123 covered by a rubber flap (not shown) permitting the introduction of liquid and preventing back flow of the same. A ventilation opening 124 is provided which is partially covered by support ribs 125 supporting an air filter (not shown).

The liquid passes down into the reception chamber 112 inside the valve body 109 via the tubular member 122. In its initial (closed) position the valve surface 115 of the valve body 109 is in contact with the valve seat 110 and consequently no liquid is allowed to pass through the holes 114. Therefore, the liquid level in the reception chamber 112 will rise and using the measuring scale 105a (see Fig. 7a) on the valve body 109, the volume of the collected liquid may be read.

When the liquid surface has reached the level of the holes 116, the introduction of additional liquid will start filling of the vessel 100.

The vessel is provided with an additional non-linear measuring scale 105b (see Fig. 7a) in the lower end of the vessel with the curved bottom surface to measure a first amount of liquid entering the vessel from the reception chamber. Due to the presence of the filling elements 106 compensating for the curved bottom surface, the measuring scale goes from the non-linear scale 105b to the linear scale 105.

If additional amounts of liquid are introduced the vessel 100 is also filled and through the hole 118 an overflow of liquid from the vessel 100 in the overflow chamber 113 may occur. From here the liquid passes via a duct 117 down through the liquid outlet 106 and into a collection bag 108.

At this time or at any earlier desired time the reception chamber 112 as well as the vessel 100 may be emptied by clockwise rotation of the wing 126. Such rotation causes the valve body 109 to be rotated and because the projection 121 is located in an upwards slanting groove (not shown) the valve body 109 is lifted upwards in connection with the rotation thus removing the valve surface 115 from the valve seat 110. Hence it is possible to empty the reception chamber quickly through the holes 114 and the vessel 100 through the space between the valve surface 115 and the valve seat 110 as liquid flows down into the collection bag 108 being connected to the outlet.

Figs. 10a-c show embodiments of a hose connection forming part of the invention as claimed, i.e. comprising a first tubular member 1 defining an outlet 2 and having a protrusion 3 provided on its outer surface. The first tubular member 1 forms part of a bottom part 4 of e.g. a vessel as described above. A semicircular section 50 partly encircles the first tubular member shown adjacent to the bottom part 4. Section 50 forms a ramp 51 having a surface for engaging with an engaging surface of the second tubular member (not shown), the surface being substantially level with the bottom part 4 at a position e.g. above the protrusion 3 and "climbing" to a level distanced from the bottom part 4 at opposite positions of the outer surface of the first tubular member 1. The semicircular section can also be level with the bottom part 4 at any other suitable position around the tubular member.

## Claims

1. A collection bag arrangement for collection of liquid from a patient comprising a hose connection, said hose connection comprising;
- a first tubular member (1) to be in fluid connection with an organ of the patient and having a longitudinal axis and one or more protrusions (3) arranged on an outer surface (22) surrounding said axis, said protrusions comprising a support surface (21) at least partly formed as a ledge, and an outlet (2), and
- a collection bag (108) having an Inlet to be connected to said outlet (2) for collection of liquid coming through said first tubular member (1), said inlet comprising a second tubular member (5) being connected to the bag (108) and adapted to receive and surround at least a part of said first tubular member (1), the second tubular member (5) having one or more recesses and/or edges (6,11) being engageable with said protrusions (3), respectively, for providing a locked separable connection between the first and second tubular member (1,5),
wherein the support surface (21) is level or flat and extends substantially perpendicularly and radially out from the outer surface (22),
wherein the first and second tubular members (1,5) are connectable by axially pushing the second tubular member (5) over the first tubular member (1) until the protrusion (3) engages the recess (6), and
wherein the first tubular member (1) and/or the second tubular member (5) comprise(s) a substantially semicircular section (50) or annular section (50) forming a ramp (51) in the axial direction engaging with said other member (1,5)

2. A collection bag arrangement according to claim 1, wherein an inclined surface (23) extends from the distal end of said support surface towards the outer surface (22).

3. A collection bag arrangement according to claim 2, wherein the inclined surface (23) has a convex form.

4. A collection bag arrangement according to any of claim 1-3, wherein the recess(es) (6) goes through the sidewall of the second member (5) and has an outline substantially identical to the outline of the protrusion(s) (3).

5. A collection bag arrangement according to any of claims 1-4, wherein the first tubular member (1) is substantially conical with an outer diameter (d3) near the outlet (2) being smaller than an outer diameter (d1) upstream thereof..

6. A collection bag arrangement according to claim 5, wherein an inner diameter (d4) of the second tubular member (5) is larger than the outer diameters (d1) and (d3) of the first tubular member (1).

7. A collection bag arrangement according to claim 6, wherein the first tubular member (1) comprises an intermediate widened portion (20) with a diameter (d2) being larger than (d1), (d3) and (d4).

8. A collection bag arrangement according to claim 7, wherein the widened portion (20) is provided by a conical protrusion on the outer surface.

9. A collection bag arrangement according to any of the preceding claims, wherein the protrusion(s) (3) are provided at a distal end of the first tubular member (1) and being adapted to engage a distal edge of the second tubular member (5).

10. A collection bag arrangement according to any of the preceding claims, wherein the protrusions (3) and recesses (6) are adapted to be disengaged by turning the second tubular member (5).

11. A collection bag arrangement according to any of the preceding claims, wherein the first tubular member (1) comprises at least two opposite flexible leg parts (10) each having a protrusion (3), the leg parts (10) being bendable towards each other for disengaging the protrusions (3) from the recess(es) (6) or edges (11) of the second tubular member (5).

12. A collection bag arrangement according to any of the preceding claims, wherein the first tubular member (1) is made of a substantially hard plastic material.

13. A collection bag arrangement according to any of the preceding claims, wherein the second tubular member (5) is made of a substantially soft plastic material.

14. A collection bag arrangement according to any of the preceding claims, wherein the first tubular member (1) is connected to a measuring vessel (100) for measuring the volume of liquid.

15. A collection bag arrangement according to claim 16, wherein the first tubular member (1) is a fixed in-moulded part of said vessel (100) and defining the outlet (104) thereof.

16. A collection bag arrangement according to any of claims 1-17, wherein the first tubular member (1) comprises two protrusions (3) arranged diametrically opposite.

17. An arrangement for measuring the volume of liquid from a patient, the arrangement comprising a measuring vessel (100) and a collection bag (108) being connected by a hose connection according to any of claims 1-16.

18. An arrangement for measuring and collecting body liquids, the arrangement comprising a measuring vessel (100) having at its top end a liquid inlet (102) and at its lower end a liquid outlet (1,104) provided with a valve and a liquid collection bag (108) connected to the liquid outlet (1,104) and suspended from the measuring vessel, a hollow valve body (109) being placed in and vertically displaceable within the measuring vessel (100), and a part of the liquid outlet has the shape of a valve seat (110) for the hollow valve body (109), the interior of the valve body being divided into a reception chamber (112) and an overflow chamber (113), said reception chamber (112) being connected to the liquid inlet (102) and having at the lower end of the valve body at least one outlet opening (114) which in the closed position of the valve body (109) is closed by the valve seat (110) and which at its upper part is connected to the measuring vessel (100) through at least one hole (116) in the chamber wall, and said overflow chamber (113) having at its lower end a duct (117) which is directly connected to the liquid outlet (1,104) of the measuring vessel (100) and which at its top end is connected to the measuring vessel (100) via a hole (118) in the chamber wall, the holes connecting the reception chamber (112) to the measuring vessel (100) and the measuring vessel (100) to the overflow chamber (113) being placed on the same side of the hollow valve body (109), and wherein the measuring vessel (100) and collection bag (108) is connected by a hose connection according to claims 1-16.

## Patentansprüche

1. Sammelbeutelanordnung zum Sammeln von Flüssigkeit von einem Patienten, umfassend eine Schlauchverbindung, wobei die Schlauchverbindung umfasst
- ein erstes schlauchförmiges Element (1) in Fluidverbindung mit einem Organ des Patienten und mit einer Längsachse und einem oder mehreren Vorsprüngen (3), die an der Außenfläche (22) um die Achse herum angeordnet sind, wobei die Vorsprünge eine Trägerfläche (21) aufweisen, die mindestens teilweise als Absatz ausgebildet ist, und mit einem Auslass (2), und
- einen Sammelbeutel (108) mit einem Einlass, der zum Sammeln von Flüssigkeit, die durch das erste schlauchförmige Element (1) strömt, mit dem Auslass (2) verbunden werden kann, wobei der Einlass ein zweites schlauchförmiges Element (5) umfasst, das mit dem Beutel (108) verbunden ist und derart ausgebildet ist, dass mindestens ein Teil des ersten schlauchförmigen Elements (1) aufgenommen und umgeben wird, wobei das zweite schlauchförmige Element (5) eine oder mehrere Aussparungen und/oder Kanten (6,11) aufweist, die jeweils mit den Vorsprüngen (3) in Eingriff gebracht werden können, um eine fixierte, trennbare Verbindung zwischen dem ersten und zweiten schlauchförmigen Element (1,5) bereitzustellen,
wobei die Trägerfläche (21) eben oder flach ist und sich im Wesentlichen senkrecht und radial nach außen von der Außenfläche (22) erstreckt,
wobei das erste und zweite schlauchförmige Element (1,5) durch Schieben in gerader Richtung des zweiten schlauchförmigen Elements (5) über das erste schlauchförmige Element (1) bis zum Eingreifen des Vorsprungs (3) in die Aussparung (6) verbunden werden können und
wobei das erste schlauchförmige Element (1) und/oder das zweite schlauchförmige Element (5) einen im Wesentlichen halbkreisförmigen Abschnitt (50) oder kreisförmigen Abschnitt (50) umfasst bzw. umfassen, der eine Rampe (51) in axialer Richtung bildet und mit dem anderen Element (1,5) in Eingriff gebracht wird.

2. Sammelbeutelanordnung nach Anspruch 1, wobei sich eine geneigte Fläche (23) vom distalen Ende der Trägerfläche zur Außenfläche (22) erstreckt.

3. Sammelbeutelanordnung nach Anspruch 2, wobei die geneigte Fläche (23) konvexe Form aufweist.

4. Sammelbeutelanordnung nach einem der Ansprüche 1-3, wobei die Aussparung(en) (6) durch die Seitenwand des zweiten Elementes (5) reicht bzw. reichen und eine Kontur aufweist bzw. aufweisen, die mit der Kontur des Vorsprungs bzw. der Vorsprünge (3) identisch ist.

5. Sammelbeutelanordnung nach einem der Ansprüche 1-4, wobei das erste schlauchförmige Element (1) im Wesentlichen konisch ist und einen Außendurchmesser (d3) nahe des Auslasses (2) aufweist der kleiner ist als ein sich stromaufwärts befindlicher Außendurchmesser (d1).

6. Sammelbeutelanordnung nach Anspruch 5, wobei ein Innendurchmesser (d4) des zweiten schlauchförmigen Elements (5) größer ist als die Außendurchmesser (d1) und (d3) des ersten schlauchförmigen Elements (1).

7. Sammelbeutelanordnung nach Anspruch 6, wobei das erste schlauchförmige Element (1) einen verbreiterten Zwischenabschnitt (20) mit einem Durchmesser (d2) umfasst, der größer ist als (d1), (d3) und (d4).

8. Sammelbeutelanordnung nach Anspruch 7, wobei der verbreiterte Zwischenabschnitt (20) durch einen konischen Vorsprung an der Außenfläche bereitgestellt ist.

9. Sammelbeutelanordnung nach einem der vorhergehenden Ansprüche, wobei der Vorsprung bzw. die Vorsprünge (3) an einem distalen Ende des ersten schlauchförmigen Elements (1) bereitgestellt ist bzw. sind und derart ausgebildet ist bzw. sind, dass ein Eingriff mit einer distalen Kante des zweiten schlauchförmigen Elements (5) bereitgestellt wird.

10. Sammelbeutelanordnung nach einem der vorhergehenden Ansprüche, wobei die Vorsprünge (3) und Aussparungen (6) derart ausgebildet sind, dass sie durch Drehen des zweiten schlauchförmigen Elements (5) getrennt werden.

11. Sammelbeutelanordnung nach einem der vorhergehenden Ansprüche, wobei das erste schlauchförmige Element (1) mindestens zwei gegenüberliegende biegsame Beinteile (10) mit jeweils einem Vorsprung (3) aufweist, wobei die Beinteile (10) gegeneinander gebogen werden können, um die Vorsprünge (3) von der Aussparung bzw. den Aussparungen (6) oder Kanten (11) des zweiten schlauchförmigen Elements (5) zu trennen.

12. Sammelbeutelanordnung nach einem der vorhergehenden Ansprüche, wobei das erste schlauchförmige Element (1) aus einem im Wesentlichen harten Kunststoffmaterial hergestellt ist.

13. Sammelbeutelanordnung nach einem der vorhergehenden Ansprüche, wobei das zweite schlauchförmige Element (5) aus einem im Wesentlichen weichen Kunststoffmaterial hergestellt ist.

14. Sammelbeutelanordnung nach einem der vorhergehenden Ansprüche, wobei das erste schlauchförmige Element (1) mit einem Messerbehälter (100) zum Messen des Flüssigkeitsvolumens verbunden ist.

15. Sammelbeutelanordnung nach Anspruch 16, wobei das erste schlauchförmige Element (1) ein fester eingeformter Teil des Behälters (100) ist und dessen Auslass (104) festlegt.

16. Sammelbeutelanordnung nach einem der Ansprüche 1-17, wobei das erste schlauchförmige Element (1) zwei Vorsprünge (3) umfasst, die diametral gegenüber angeordnet sind.

17. Anordnung zum Messen des Flüssigkeitsvolumens eines Patienten, wobei die Anordnung einen Messbehälter (100) und einen Sammelbeutel (108) umfasst, die durch eine Schlauchverbindung nach einem der Ansprüche 1-16 verbunden sind.

18. Anordnung zum Messen und Sammeln von Körperflüssigkeiten, wobei die Anordnung einen Messbehälter (100) umfasst, der an seinem oberen Ende einen Flüssigkeitseinlass (102) und an seinem unteren Ende einen Flüssigkeitsauslass (1, 1046) aufweist, der mit einem Ventil und einem Flüssigkeitssammelbeutel (108) versehen ist, der mit dem Flüssigkeitsauslass (1, 104) verbunden ist und vom Messbehälter hängt, wobei ein hohles Ventilgehäuse (109) im Messbehälter (100) angeordnet und darin vertikal verschiebbar ist, und wobei ein Teil des Flüssigkeitsauslasses die Form eines Ventilsitzes (110) für das hohle Ventilgehäuse (109) aufweist, wobei das Innere des Ventilgehäuses in eine Aufnahmekammer (112) und eine Überlaufkammer (113) unterteilt ist, wobei die Aufnahmekammer (112) mit dem Flüssigkeitseinlass (102) verbunden ist und am unteren Ende des Ventilgehäuses wenigstens eine Auslassöffnung (114) aufweist, die in der geschlossenen Stellung des Ventilgehäuses (109) durch den Ventilsitz (110) geschlossen ist, und die an ihrem oberen Ende über wenigstens eine Öffnung (116) in der Kammerwand mit dem Messbehälter (100) verbunden ist, und wobei die Überlaufkammer (113) an ihrem unteren Ende einen Kanal (117) aufweist, der direkt mit dem Flüssigkeitsauslass (1, 104) des Messbehälters (100) verbunden ist und die an ihrem oberen Ende über eine Öffnung (118) in der Kammerwand mit dem Messbehälter (100) verbunden ist, wobei die Öffnungen, die die Aufnahmekammer (112) mit dem Messbehälter (100) und den Messbehälter (100) mit der Überlaufkammer (113) verbinden, auf derselben Seite des hohlen Ventilgehäuses (109) angeordnet sind, und wobei der Messbehälter (100) und der Sammelbeutel (108) über eine Schlauchverbindung nach Anspruch 1-16 verbunden sind.

## Revendications

1. Système de poche de recueil destiné à recueillir un liquide provenant d'un patient comprenant un raccordement à tubulure, ledit raccordement à tubulure comprenant :
- une première pièce tubulaire (1) destinée à être raccordée à un fluide d'un organe du patient et possédant un axe longitudinal et une ou plusieurs protubérances (3) disposées sur une surface externe (22) entourant ledit axe, lesdites protubérances comprenant une surface de support (21) formant au moins en partie un rebord, et une sortie (2), et
- une poche de recueil (108) possédant une entrée à raccorder à ladite sortie (2) pour recueillir un liquide arrivant par ladite première pièce tubulaire (1), ladite entrée comprenant une deuxième pièce tubulaire (5) étant raccordée à la poche (108) et adaptée pour recevoir et entourer au moins une partie de ladite première pièce tubulaire (1), la deuxième pièce tubulaire (5) comportant un ou plusieurs évidements et/ou bords (6, 11) dans lesquels lesdites protubérances (3) peuvent être respectivement engagées pour former un raccord séparable verrouillé entre la première et la deuxième pièce tubulaire (1, 5),
où la surface de support (21) est de niveau ou plate et s'étend essentiellement perpendiculairement et radialement hors de la surface externe (22),
où la première et la deuxième pièce tubulaire (1, 5) peuvent être raccordées en poussant axialement la deuxième pièce tubulaire (5) par-dessus la première pièce tubulaire (1) jusqu'à ce que la protubérance (3) s'engage dans l'évidement (6), et
où la première pièce tubulaire (1) et/ou la deuxième pièce tubulaire (5) comprennent une section essentiellement semi-circulaire (50) ou une section annulaire (50) formant une rampe (51) dans le sens axial s'engageant dans l'autre pièce (1, 5).

2. Système de poche de recueil selon la revendication 1, où une surface inclinée (23) s'étend depuis l'extrémité distale de ladite surface de support vers la surface externe (22).

3. Système de poche de recueil selon la revendication 2, où la surface inclinée (23) possède une forme convexe.

4. Système de poche de recueil selon l'une quelconque des revendications 1 à 3, où le(s) évidement(s) (6) traverse(nt) la paroi latérale de la deuxième pièce (5) et présente(nt) un contour essentiellement identique au contour de la(des) protubérance(s) (3).

5. Système de poche de recueil selon l'une quelconque des revendications 1 à 4, où la première pièce tubulaire (1) est essentiellement conique avec un diamètre externe (d3) à proximité de la sortie (2) inférieur au diamètre externe (d1) en amont de celle-ci.

6. Système de poche de recueil selon la revendication 5, où un diamètre interne (d4) de la deuxième pièce tubulaire (5) est supérieur aux diamètres externes (d1) et (d3) de la première pièce tubulaire (1).

7. Système de poche de recueil selon la revendication 6, où la première pièce tubulaire (1) comprend une partie élargie intermédiaire (20) de diamètre (d2) supérieur à (d1), (d3) et (d4).

8. Système de poche de recueil selon la revendication 7, où la partie élargie (20) est formée par une protubérance conique sur la surface externe.

9. Système de poche de recueil selon l'une quelconque des revendications précédentes, où la(les) protubérance(s) (3) se trouvent à une extrémité distale de la première pièce tubulaire (1) et sont adaptées pour s'engager dans un bord distal de la deuxième pièce tubulaire (5).

10. Système de poche de recueil selon l'une quelconque des revendications précédentes, où les protubérances (3) et les évidements (6) sont adaptés pour être dégagés en faisant tourner la deuxième pièce tubulaire (5).

11. Système de poche de recueil selon l'une quelconque des revendications précédentes, où la première pièce tubulaire (1) comprend au moins deux pattes flexibles opposées (10) possédant chacune une protubérance (3), les pattes (10) pouvant être inclinées l'une vers l'autre pour dégager les protubérances (3) du(des) évidement(s) (6) ou bords (11) de la deuxième pièce tubulaire (5).

12. Système de poche de recueil selon l'une quelconque des revendications précédentes, où la première pièce tubulaire (1) est constituée d'une matière plastique essentiellement dure.

13. Système de poche de recueil selon l'une quelconque des revendications précédentes, où la deuxième pièce tubulaire (5) est constituée d'une matière plastique essentiellement souple.

14. Système de poche de recueil selon l'une quelconque des revendications précédentes, où la première pièce tubulaire (1) est raccordée à un récipient de mesure (100) pour mesurer le volume de liquide.

15. Système de poche de recueil selon la revendication 16, où la première pièce tubulaire (1) est une partie fixe moulée dans ledit récipient (100) et définit la sortie (104) de celui-ci.

16. Système de poche de recueil selon l'une quelconque des revendications 1 à 17, où la première pièce tubulaire (1) comprend deux protubérances (3) situées en des points diamétralement opposés.

17. Système de mesure du volume de liquide provenant d'un patient, le système comprenant un récipient de mesure (100) et une poche de recueil (108) qui est raccordée par un raccordement à tubulure selon l'une quelconque des revendications 1 à 16.

18. Système de mesure et de recueil de liquides corporels, le système comprenant un récipient de mesure (100) possédant à son extrémité supérieure une entrée de liquide (102) et à son extrémité inférieure une sortie de liquide (1, 104) munie d'une valve et d'une poche de recueil de liquide (108) raccordée à la sortie de liquide (1, 104) et suspendue au récipient de mesure, un corps de valve creux (109) étant placé et verticalement déplaçable dans le récipient de mesure (100), et une partie de la sortie de liquide étant en forme de siège de valve (110) pour le corps de valve creux (109), l'intérieur du corps de valve étant divisé en une chambre réceptrice (112) et une chambre de trop-plein (113), ladite chambre réceptrice (112) étant raccordée à l'entrée de liquide (102) et possédant à l'extrémité inférieure du corps de valve au moins une ouverture de sortie (114) qui, quand le corps de valve (109) est en position fermée, est fermée par le siège de valve (110) et qui, au niveau de sa partie supérieure, est raccordée au récipient de mesure (100) au travers d'au moins un trou (116) dans la paroi de la chambre, et ladite chambre de trop-plein (113) possédant à son extrémité inférieure un conduit (117) qui est directement raccordé à la sortie de liquide (1, 104) du récipient de mesure (100) et qui, à son extrémité supérieure, est raccordé au récipient de mesure (100) par un trou (118) dans la paroi de la chambre, les trous reliant la chambre réceptrice (112) au récipient de mesure (100) et le récipient de mesure (100) à la chambre de trop-plein (113) étant situés du même côté du corps de valve creux (109), et où le récipient de mesure (100) et la poche de recueil (118) sont raccordés par un raccordement à tubulure selon les revendications 1 à 16.
